# EUROPEAN PATENT APPLICATION

(11) **EP 2 599 469 A1**
(43) Date of publication of application: **05.06.2013**
(21) Application number: 11812389.2
(22) Date of filing: 22.07.2011
(51) Int. Cl.: A61H 39/00, A61N 5/06

(54) **PAIN-RELIEF DEVICE**

(30) Priority: 28.07.2010 JP 2010168751
(71) Applicant: YA-MAN LTD, Koto-ku, Tokyo 135-0045 (JP)
(72) Inventor: YAMAZAKI, Akitsugu, Tokyo 135-0045 (JP); YAMAZAKI, Iwao, Tokyo 135-0045 (JP)
(74) Representative: Emerson, Peter James
(86) International application number: PCT/JP2011/066679
(87) International publication number: WO 2012/014799

(57) **Abstract**

Existing pain-relief devices have the problem that the use thereof requires specialized knowledge of Eastern acupuncture, e.g. meridians and acupuncture points, making it difficult for ordinary people to use said devices to relieve pain in painful areas. The disclosed pain-relief device is provided with: a small camera that, in step ST3, takes a picture of a painful area and outputs a camera signal; a camera-signal processing circuit that processes the camera signal from the small camera; a display unit that, in step ST4, displays an image of the painful area, obtained via the processing of the camera signal in the camera-signal processing circuit; and a semiconductor laser that, in step ST7, is driven by a laser-driving circuit and irradiates the painful area with heat-producing laser light.

## Description

### TECHNICAL FIELD

The present invention relates to a pain-relief device which applies a laser light to relieve pain in painful areas.

### BACKGROUND ART

FIG. 9 is a block diagram of a conventional pain-relief device which is disclosed in Patent Document 1.

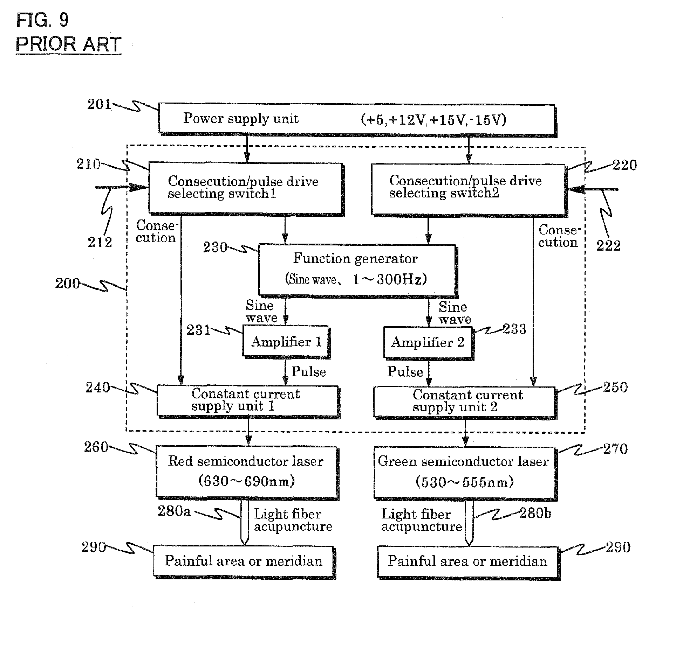

In FIG.9, a driving circuit is denoted by 200, a power supply unit is denoted by 201, a consecution/pulse drive selecting switch are denoted by 210 and 220, a function generator is denoted by 230, amplifiers are denoted by 231 and 233, constant current supply units are denoted by 240 and 250, a red semiconductor laser is denoted by 260, a green semiconductor laser is denoted by 270, light fiber acupunctures are denoted by 280a and 280b, and a painful area or a meridian is denoted by 290.

The light fiber acupunctures 280a and 280b have been developed to produce both a metal acupuncture function of Eastern acupuncture and a laser light irradiation. A thin metallurgical coating is applied to a clad of the light fiber whose jacket or trunking has been removed and a surface fabrication is applied at a predetermined angle to its end.

Next, the operation will be explained.
First the consecution mode or the pulse mode is selected at the consecution/pulse drive selecting switches 210 and 220 by way of control signals 212 and 222. Subsequently the red semiconductor laser 260 or the green semiconductor laser 270 produces the consecutive oscillation or the pulse oscillation to emit laser light by the functions of the function generator 230, the amplifiers 231 and 233, and the constant current supply units 240 and 250. The laser light emitted by the red semiconductor laser 260 or the green semiconductor laser 270 irradiates the painful area or the meridian 290 through the light fiber acupunctures 280a and 280b.

### PRIOR ART

Patent Document 1:
Japanese Patent Application Laid-Open No. 2010-12268
Patent Document 2:
Japanese Patent Application Laid-Open No. 2004-329474

### SUMMARY OF THE INVENTION

### TECHNICAL PROBLEMS

An existing pain-relief device requires expert knowledge of Eastern acupuncture of a user, such as meridians and acupuncture points. Accordingly, there has been a problem that it is difficult for ordinary people to use it to relieve pain in painful areas.

Such is the case with the non-invasive pain-relief device such as the one disclosed in Japanese Patent Application Laid-Open No. 2004-329474 (Patent Document 2) as well as the invasive pain-relief device. Laser acupuncture which contributes to effective treatment by collecting laser light into acupuncture points to apply a stimulus by means of a light collecting mechanism or the like also requires expert knowledge of acupuncture of a user, making it difficult for ordinary people to use it.

In order to solve the problems described above, the present invention allows one to use the pain-relief device without expert knowledge of acupuncture so that ordinary people (i.e. an acupuncture inexpert) can use it to relieve pain in painful areas without difficulty.

### SOLUTION TO PROBLEM

The pain-relief device according to claim 1 of the present invention includes some mechanisms as follows: a laser light source mechanism which emits heat-producing laser light; an image-taking mechanism which takes a picture of a painful area; and an image display mechanism which displays the image of the painful area taken by said image-taking mechanism. The painful area which has been imaged by said image-taking mechanism is irradiated with said laser light.

In addition to above described respective mechanism as in claim 1, the pain-relief device according to claim 2 further includes a control mechanism which controls the laser light source mechanism based on control-relevant information of the laser light source mechanism, and a control information presenting mechanism which presents the control-relevant information to the user.

Moreover, in addition to above described respective mechanism as in claim 1, the pain-relief device according to claim 3 further includes an emission request mechanism which is utilized for the laser light emission from the laser light source mechanism. The laser light source mechanism emits the laser light when the emission request mechanism is used.

With the pain-relief device according to one embodiment of claim 4, the image-taking mechanism as in claim 1 is further provided with an illuminating mechanism which illuminates the painful areas.

In addition to above described respective mechanism as in claim 1, the pain-relief device according to claim 5 further includes a touch sensor mechanism installed around an irradiation part wherein the painful area is irradiated with the laser light to sense a contact with the painful area and a control mechanism which controls the laser light source mechanism to emit the laser light only while the touch sensor mechanism is contacting the painful area.

Furthermore, in addition to above described respective mechanism as in claim 1, the pain-relief device according to claim 6 further includes a light diffusion mechanism which has a negative refracting power and irradiates the painful areas with the laser light emitted from the laser light source mechanism.

With the pain-relief device according to one embodiment of claim 7, the laser light source mechanism as in claim 1 is a semiconductor laser.

With the pain-relief device according to one embodiment of claim 8, the light source mechanism as in claim 1 emits the laser light to the painful area within a range of 0.4 W/cm² or less in power.

With the pain-relief device according to one embodiment of claim 9, the light source mechanism as in claim 1 emits the laser light to the painful area within a range from 600nm to 1000nm in wavelength.

### ADVANTAGEOUS EFFECTS OF INVENTION

As described above, since claim 1 of the present invention is provided with the laser light source mechanism, it becomes possible to relieve the pain of a bruise, sprain, shoulder stiffness, backache or the like by irradiating the painful area with the heat-producing laser light so as to warm the painful area and to promote blood circulation. Moreover, due to the image-taking mechanism which takes a picture of the painful area and the image display mechanism which displays the image of the painful area taken by said image-taking mechanism, the painful area imaged by the image-taking mechanism is to be irradiated with the laser light, making it possible to roughly narrow the area being laser-irradiated by checking anomalies within the painful area such as reddening, roughness, and/or dryness of skin or the like. Accordingly, it becomes possible for ordinary people without expert knowledge of acupuncture to provide a treatment to relieve pain without difficulty.

As claim 2 of the present invention further includes a control mechanism which controls the laser light source mechanism based on control-relevant information of the laser light source mechanism and a control information presenting mechanism which presents the control-relevant information to the user, the user can get a handle on the operation i.e. the improvement of the operability of the pain-relief device can be achieved, in addition to the efficacy achieved in claim 1.

As claim 3 of the present invention further includes an emission request mechanism which is utilized for the laser light emission from the laser light source mechanism and the laser light source mechanism emits the laser light when the emission request mechanism is used, it becomes possible to control emission/non-emission of the laser light without difficulty, resulting in the improvement of the operability of the pain-relief device. Also in addition to the efficacy achieved in claim 1, since the laser light is not emitted unless the emission request mechanism is used, an erroneous irradiation of the laser light can be prevented so as to avoid accidents causing blindness or the like, resulting in the safety enhancement of the pain-relief device.

In addition to the efficacy achieved in claim 1, since the image-taking mechanism as in claim 4 of the present invention further includes an illuminating mechanism which illuminates the painful areas, it becomes possible to reliably take a picture in bright light even when there is insufficient light volume, resulting in the capability of checking anomalies within the painful areas.

Claim 5 of the present invention may further includes a touch sensor mechanism installed around the irradiation part wherein the painful area is irradiated with the laser light to sense a contact with the painful area and the control mechanism which controls the laser light source mechanism to emit the laser light only while the touch sensor mechanism is contacting the painful area. Accordingly, in addition to the efficacy achieved in claim 1, since the laser light is not emitted from the laser light source mechanism when the irradiation part is not near the painful area i.e. because there is no contact between the touch sensor mechanism and the painful area, it becomes possible to avoid erroneous irradiation of the laser light or accidents causing blindness or the like, resulting in the safety enhancement of the pain-relief device.

In addition to the efficacy achieved in claim 1, as the claim 6 of the present invention further includes the light diffusion mechanism which has the negative refracting power and irradiates the painful areas with the laser light emitted from the laser light source mechanism, it becomes possible to diffuse the laser light widely, resulting in the improvement in efficiency of the irradiation. It also becomes possible to irradiate the painful area with the uniform laser light, producing the reduction of the unevenness of the irradiation. Furthermore, it also becomes possible to irradiate the painful area with the laser light having a mild laser light power.

In addition to the efficacy achieved in claim 1, since the laser light source mechanism as in claim 7 of the present invention includes a semiconductor laser which is compact, lightweight, longer lasting and low priced, the longer lasting pain-relief device which is reduced in size, weight and cost as a whole can be achieved.

In addition to the efficacy achieved in claim 1, as the laser light source mechanism as in claim 8 of the present invention is characterized in that the laser light emitted to the painful area has a power within a range of 0.4 W/cm² or less, the painful area is irradiated with the laser light within said range of power, making it possible to warm the painful area and to promote blood circulation so as to relieve pain.

In addition to the efficacy achieved in claim 1, as the laser light source mechanism as in claim 9 of the present invention is characterized in that the laser light emitted to the painful area is ranged from 600nm to 1000nm in wavelength, the painful area is irradiated with the laser light within said range of wavelength, making it possible to warm the painful area and to promote blood circulation so as to relieve pain.

### BRIEF DESCRIPTION OF THE DRAWINGS

FIG. 1 is an appearance diagram of the pain-relief device of the present invention.
FIG. 2 is a configuration block diagram of the pain-relief device according to the first embodiment of the present invention.
FIG. 3 is a flow chart which illustrates the operation of the pain-relief device according to the first embodiment of the present invention.
FIG. 4 is a configuration block diagram of the pain-relief device according to the second embodiment of the present invention.
FIG. 5 is a flow chart which illustrates the operation of the pain-relief device according to the second embodiment of the present invention.
FIG. 6 is a configuration block diagram of the pain-relief device according to the third embodiment of the present invention.
FIG. 7 is a flow chart which illustrates the operation of the pain-relief device according to the third embodiment of the present invention.
FIG. 8 is a configuration block diagram of the pain-relief device according to the fourth embodiment of the invention.
FIG. 9 is a configuration diagram of the conventional pain-relief device in the prior art.

### DETAILED DESCRIPTION OF THE INVENTION

Hereinafter, embodiments of this invention will be concretely described with reference to the drawings. In each of the drawings, same numerical symbols are used to the same constitutions or corresponding constitutions.

### EMBODIMENT 1

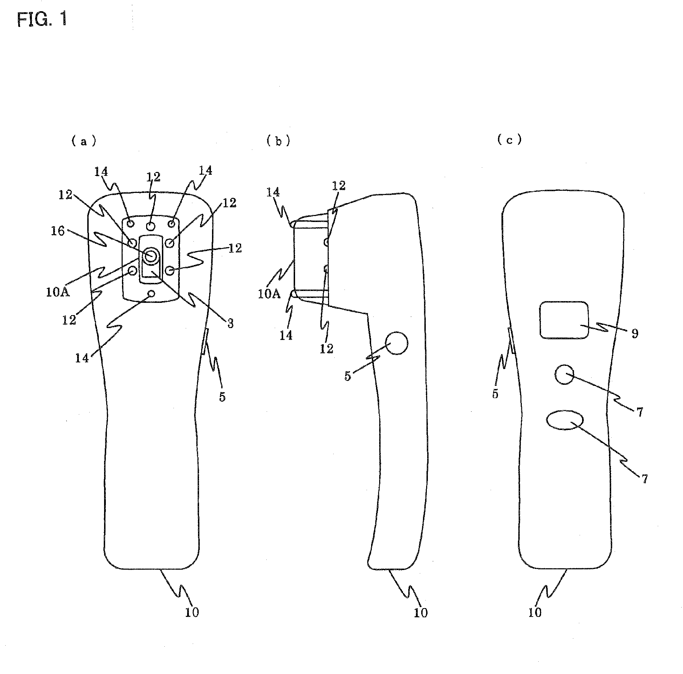

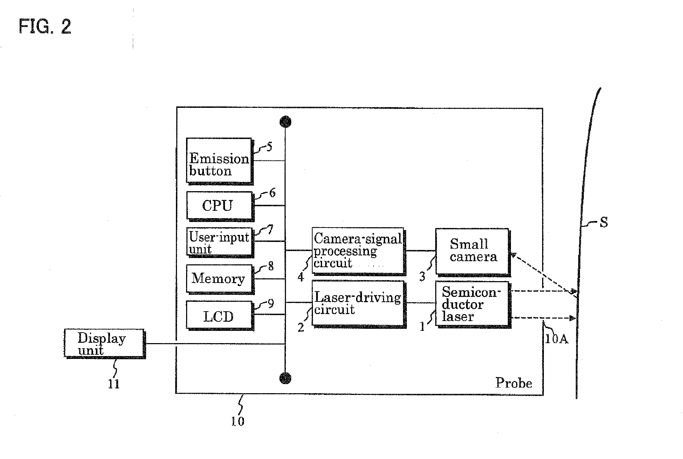

FIG. 1 is an appearance diagram of the pain-relief device of the present invention. FIG. 1(a), (b) and (c) are a front view, a lateral view and a back view respectively. FIG. 2 is a configuration block diagram of the pain-relief device according to the first embodiment of the present invention.

In FIG. 1 and FIG. 2, a painful area is denoted by S, a semiconductor laser which emits relatively low-powered heat-producing laser light is denoted by 1, a laser-driving circuit which drives the semiconductor laser 1 is denoted by 2, a small camera which takes a picture of the painful area S by mechanism of image sensors such as CCD (*1) or CMOS (*2) is denoted by 3, and a camera-signal processing circuit which processes the image taken by the small camera 3 is denoted by 4. The laser light source mechanism is included of the semiconductor laser 1 and the laser-driving circuit 2, and the image-taking mechanism is included of the small camera 3 and the camera-signal processing circuit 4, respectively.

(*1) CCD is an abbreviation of Charge Coupled Device.
(*2) CMOS is an abbreviation of Complementary Metal Oxide Semiconductor.

Further, in FIG. 1 and FIG. 2, an emission button as an emission request mechanism which is switched ON when the laser light is emitted from the semiconductor laser 1 is denoted by 5; a CPU as a control mechanism which controls the pain-relief device is denoted by 6; a user-input unit by which information is input to the pain-relief device is denoted by 7; a memory such as ROM or RAM is denoted by 8; LCD (*3) as a control information presenting mechanism which presents information to a user is denoted by 9; a portable probe is denoted by 10; an irradiation part of the probe where the laser light irradiated to the painful area S transits therein is denoted by 10A; and a display unit as an external image display mechanism is denoted by 11.

(*3) LCD is an abbreviation of Liquid Crystal Display.

Next, the operation will be explained.

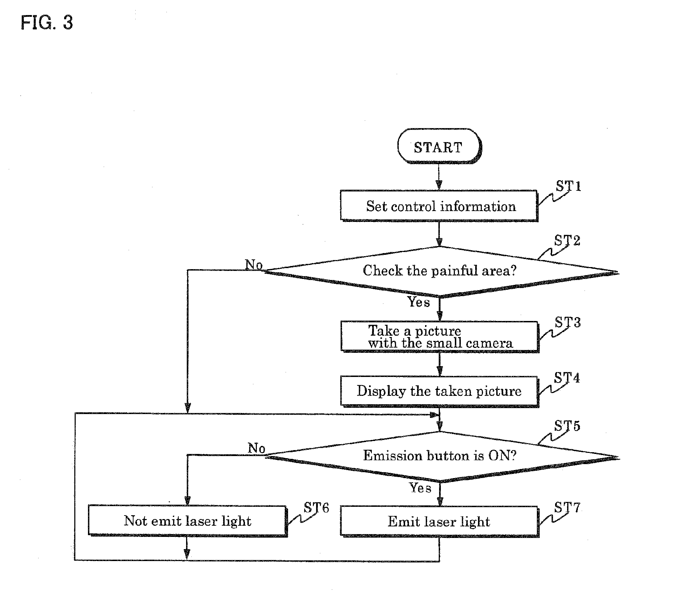

FIG. 3 is a flow chart which illustrates the operation of the pain-relief device according to the first embodiment of the present invention.
In reference to FIG. 3, when the power button on the user-input unit 7 is switched ON for power activation, the CPU 6 loads the program from the memory 8 so that the pain-relief device is activated.

First the CPU 6 sequentially displays the control information of the semiconductor laser 1 such as the power of the laser light or the irradiation time per irradiation on the LCD 9 so that the user can perceive the control information and set the intended control information by mechanism of the user-input unit 7 (Step ST1). Since the control information is displayed on LCD 9, the user can perceive the control information of the semiconductor laser 1 without difficulty, that the operability of the pain-relief device can be improved. Herein, the control information is not necessarily displayed on LCD 9. Instead it is possible to be displayed on other display mechanism, or it is possible to employ a loudspeaker or the like to present the control information to the user by way of artificial voice.

After the control information is set, the CPU 6 stands by for user input to provide directions for whether or not to check the painful area S (Step ST2). When the painful area S does not need to be checked and the user operates the user-input unit 7 to input "No" ("No" in Step ST2), it is followed by Step ST 5. On the other hand, when the painful area S need to be checked and the user operates the user-input unit 7 to input "Yes" ("Yes" in Step ST2), the CPU 6 controls the small camera 3 to take a picture of the painful area S so that the camera signal is output (Step ST 3). Then, said camera signal is processed by the camera-signal processing circuit 4 so that the image of the painful area S is displayed on the display unit 11 (Step ST4).

As previously indicated, irrespective of the type i.e. invasive or non-invasive, a conventional pain-relief device requires the user to have expert knowledge of Eastern acupuncture, making it difficult for ordinary people to use it. In contrast to this, the pain-relief device according to the first embodiment of the present invention utilizes the so-called hyper thermic effect of the low-powered laser light so that the heat-producing laser light can be diffused over a certain range to irradiate the painful area without being condensed. Accordingly, as the painful area S is warmed and the blood circulation is improved, it becomes possible to relieve pain of bruise, sprain, shoulder stiffness, backache or the like. Compared to the conventional pain-relief device, the irradiated area is wider so that the pain-relief effect can be achieved by only irradiating around the painful area.

In general, the user can visually realize anomalies within the painful area S such as roughness, reddish area or dryness of skin. Particularly an enlarged display makes it easier to determine the anomalies. Thus, the pain-relief device according to the first embodiment is included of the small camera 3 and the camera-signal processing circuit 4 so as to take a picture of the painful area S and to display the enlarged image of the taken picture on the display unit 11. Therefore, the anomalies within the painful area S such as redness, roughness, or dryness of skin can be checked through the steps ST2 to ST4.

As described above, since the anomalies within the painful area S can be checked on the visual display unit 11 so that the area being laser-irradiated can be roughly narrowed, it becomes possible for ordinary people who have no expert knowledge of acupuncture to provide a treatment to relieve pain without difficulty. In addition, the treatment can be applied to not only a human being but also an animal which does not communicate with human languages and whose body is covered with fur so that the painful area S cannot be checked practically.

Referring again to FIG. 3, after the image of the painful area S is displayed on the display unit 11 at step ST4, the CPU 6 stands by for a user input to switch ON the emission button 5 (step ST5). When the emission button 5 placed on the grip of the pain-relief device is not switched ON by the user ("No" in step ST5), the CPU 6 does not activate the laser-driving circuit 2 i.e. the laser light is not emitted from the semiconductor laser 1 (step ST6).

On the other hand, when the irradiation part 10A approaches the painful area S and the emission button 5 is switched ON by the user ("Yes" in step ST5), the CPU 6 activates the laser-driving circuit 2 so that the semiconductor laser 1 is controlled to emit the laser light based on the control information set at the step ST1 (step ST7). As previously indicated, the laser light is diffused over the certain range to irradiate the painful area S with the hyper thermic effect so that the painful area S can be warmed and the pain in the painful area S can be relieved. Thereafter, the step ST6 or the step ST7 will follow corresponding to ON/OFF of the emission button 5 (step ST5).

In this way, as the probe 10 of the pain-relief device shown in FIG. 1 and FIG. 2 is provided with the laser light emission button 5, it becomes possible to control emission/non-emission of the laser light without difficulty, resulting in the improvement of the operability of the pain-relief device.
In addition, since the laser light is not emitted from the semiconductor laser 1 unless the emission button 5 is switched ON by the user, an erroneous irradiation of the laser light can be prevented so as to avoid accidents causing blindness or the like, resulting in the safety enhancement of the pain-relief device.

With this first reference example, the laser light emission is controlled by the CPU 6 when the emission button 5 is switched ON. However, the emission request mechanism is not limited to the button-typed one. A slide-type, a lever-type, or a voice-input-type is also possible. In addition, the method is not limited to controlling the emission with the CPU 6. It is also possible to control the supply current to the semiconductor laser 1 or to control the attenuation of the laser light emitted from the semiconductor laser 1.

The processing that occurs when the CPU 6 activates the small camera 3 when input "Yes" at the step ST2 in FIG. 3 can be replaced with the following: That is, for example when the control information is set at the step ST1, it is possible that the CPU 6 activates the small camera 3 immediately and keeps taking pictures of the painful area S while the pain-relief device is running so that the display unit 11 keeps displaying the taken images thereon.

As described above, according to the first embodiment, the small camera 3 which takes a picture of the painful area S and outputs the camera signal and the camera-signal processing circuit 4 which processes the camera signal output from the small camera 3 are provided at the step ST3; the display unit 11 which displays the taken image of the painful area S obtained by processing the camera signal with the camera-signal processing circuit 4 is provided at the step ST4; and the semiconductor laser 1 which is activated by the laser-driving circuit 2 and emits the heat-producing laser light to irradiate the painful area S is provided at the step ST7. Accordingly, the anomalies within the painful area S such as redness, roughness, or dryness of skin can be checked and the area being laser-irradiated can be roughly narrowed so that the ordinary people who have no expert knowledge of acupuncture are enabled to provide a treatment to relieve pain without difficulty.

In addition, according to the first embodiment, the CPU 6 which controls the semiconductor laser 1 based on the control information thereof is by mechanism of the laser-driving circuit 2; and the LCD 9 which displays the control information thereon for the user is further provided at the step ST1. Accordingly, it becomes possible for the user to perceive the control regulation of the laser light irradiating the painful area S, resulting in the improvement of the operability of the pain-relief device.

In addition, according to the first embodiment, the emission button 5 by which the semiconductor laser 1 is made to emit the laser light to irradiate the painful area S when it is switched ON is further provided at the step ST5. Accordingly, it becomes possible to control emission/non-emission of the laser light without difficulty, resulting in the improvement of the operability of the pain-relief device. Moreover, since the laser light is not emitted from the semiconductor laser 1 unless the emission button 5 is switched ON, an erroneous irradiation of the laser light can be prevented so as to avoid accidents causing blindness or the like, resulting in the safety enhancement of the pain-relief device.

In addition, according to the first embodiment, the semiconductor laser 1 which emits the heat-producing laser light is further provided. Since the semiconductor laser 1 is compact, lightweight, longer lasting and low priced, the longer lasting pain-relief device which is reduced in size, weight and cost as a whole can be achieved.

### EMBODIMENT 2

When the image of the painful area S is taken, there is a possibility of causing a shadow or being dark around the shooting location i.e. there is a possibility of causing a deficiency of light volume. The following processing is possible to overcome such situations.

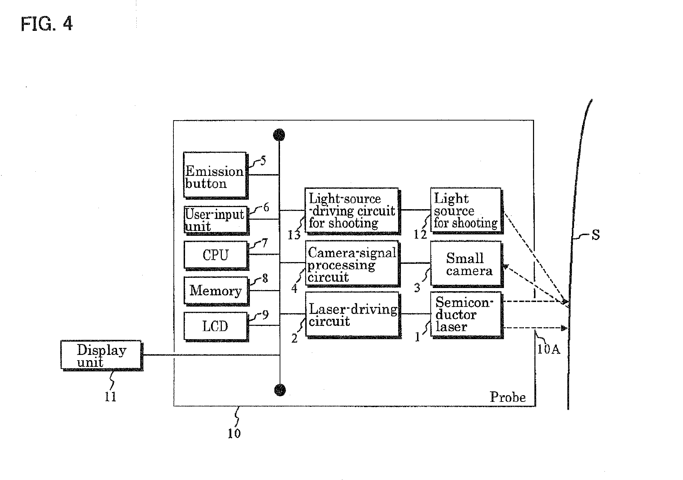

FIG. 4 is a configuration block diagram of the pain-relief device according to the second embodiment of the present invention.
In FIG. 1 and FIG. 4, a light source for shooting which illuminates the painful area S is denoted by 12, and the light-source-driving circuit for shooting which activates the light source for shooting 12 is denoted by 13. An illuminating mechanism is included of the light source for shooting 12 and the light-source-driving circuit for shooting 13.

### Next, the operation will be explained.

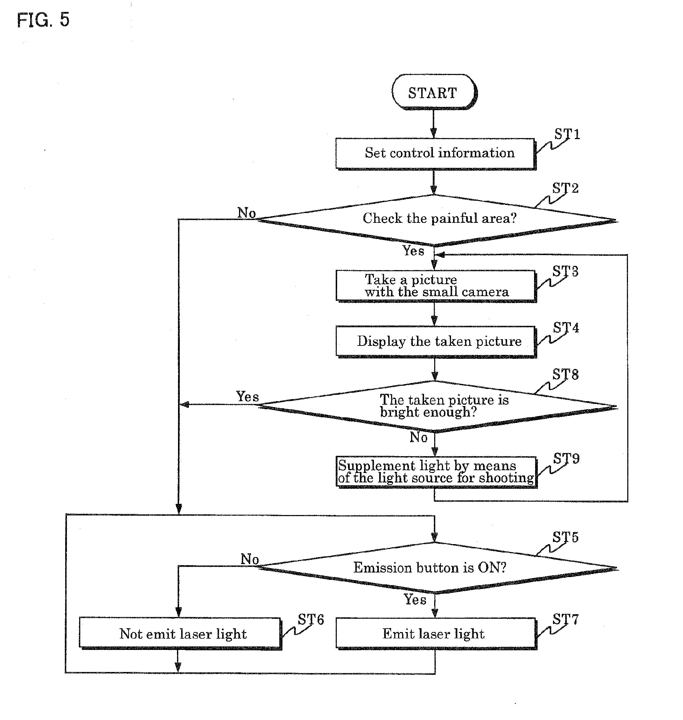

FIG. 5 is a flow chart which illustrates the operation of the pain-relief device according to the second embodiment of the present invention.
In FIGS., as well as the embodiment shown in FIG. 3, after the steps 1 to 4 the CPU 6 stands by for a user input with regard to the use of the light source for shooting 12 (step ST8).

The user checks the image of the painful area S displayed on the display unit 11. When the illumination of the image is sufficient, the user inputs "No" in the user-input unit 7 ("No" at step ST8), and the CPU 6 proceeds to the step ST5 to function the same way as in the first embodiment. On the other hand, when the illumination of the image displayed on the display unit 11 is insufficient, the user inputs "Yes" in the user-input unit 7 ("Yes" at step ST8), and the CPU 6 activates the light-source-driving circuit for shooting 13 to activate the light source for shooting 12 so that the painful area S is illuminated (step ST9).

As described above, the pain-relief device shown in FIG. 4 is provided with the light source for shooting 12 and the light-source-driving circuit for shooting 13, and the light supplement is provided when the picture is taken at the step ST9. Therefore, it becomes possible to reliably take a picture of the painful area S in bright light by supplementing light thereto even when the light volume of the shooting location is deficient, resulting in the capability of checking anomalies within the painful area S.

In this way, the image of the painful area S in bright light can be obtained. And when the user inputs "Yes" in the user-input unit 7 at the step ST8, the CPU 6 proceeds to the step ST5 to function the same way as in the first embodiment.

As above, according to the second embodiment, the light source for shooting 12 and the light-source-driving circuit for shooting 13 which illuminate the painful area S at the step ST9 are further provided so as to accommodate the case wherein the brightness level of the image of the painful area S displayed on the display unit 11 is deficient at the step ST4 i.e. when "No" at the step ST8. Accordingly, it becomes possible to reliably take a picture of the painful area S in bright light even when a light volume for shooting the painful area S with the small camera 3 is deficient, resulting in the capability of checking anomalies within the painful area S.

Herein, the pain relieving device does not necessarily have to utilize the small camera 3 which requires the light source for shooting 12 and the light-source-driving circuit for shooting 13. It is possible to utilize an infrared camera which does not require the light source for shooting 12 and the light-source-driving circuit for shooting 13. In addition, it is also possible to keep lighting the light source for shooting 12 and to keep taking pictures of the painful area S while the pain-relief device is running.

### EMBODIMENT 3

In order to avoid erroneous irradiation of the laser light which may cause blindness or the like, the following embodiment allows one to enhance the safety of the pain-relief device.

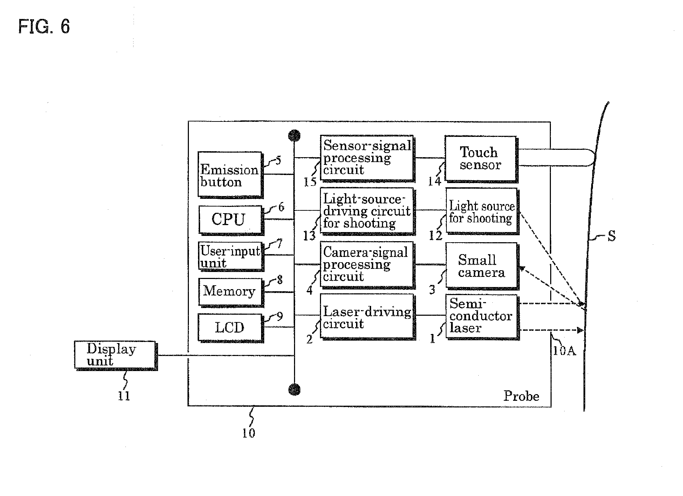

FIG. 6 is a configuration block diagram of the pain-relief device according to the third embodiment of the present invention.
In FIG. 1 and FIG. 6, 14 denotes one or more touch sensors placed around the irradiation part 10A, which outputs the sensor signal when it contacts the painful area S. 15 denotes a sensor-signal processing circuit which senses a contact between the touch sensor 14 and the painful area S by processing the sensor signal output from the touch sensor 14.
A touch sensor mechanism is included of the touch sensor 14 and the sensor-signal processing circuit 15.

In FIG. 1, three touch sensors 14 are provided surrounding the irradiation part 10A, projecting outward from the irradiation part 10A to the side of painful area S for about several millimeters in length. That is to say, when the irradiation part 10A approaches the painful area S to irradiate the painful area S with the laser light, the touch sensor 14 contacts the painful area S. Herein, the number of the touch sensors 14 is not limited to three. Any given number is possible corresponding to layouts.

### Next, the operation will be explained.

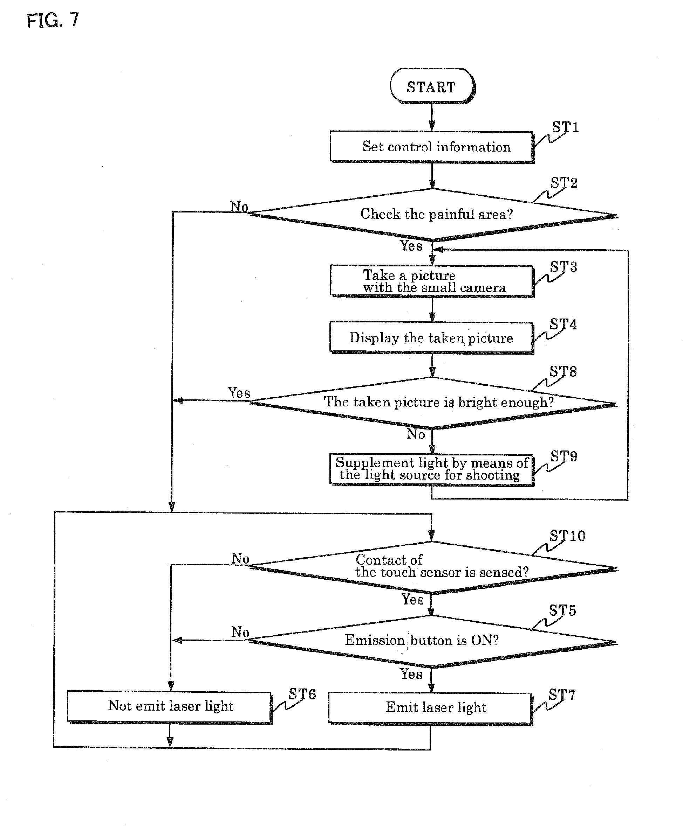

FIG. 7 is a flow chart which illustrates the operation of the pain-relief device according to the third embodiment of the present invention.
In FIG. 7, in the same way as in FIG. 5, after the steps ST1 to ST4, ST8 and ST9, the CPU 6 determines the contact between the touch sensor 14 and the painful area S by mechanism of the sensor-signal processing circuit 15 (step ST10). When no contact has been sensed by the sensor-signal processing circuit 15 (when "No" at the step ST10), the CPU 6 determines that the irradiation part 10A is not approaching the painful area S so as not to activate the laser-driving circuit 2 to emit the laser light from the semiconductor laser 1, and goes into standby mode to prevent erroneous irradiation ("No" at the step ST10 ∼ step ST6).

While in the standby mode, when the touch sensor 14 contacts the painful area S to output the sensor signal so that the contact is sensed by the sensor-signal processing circuit 15 ("Yes" at the step ST10), the CPU 6 determines that the irradiation part 10A is approaching the painful area S and determines whether or not the emission button 5 is switched ON in the same way as in the first embodiment (step ST5). When the emission button 5 is not switched ON ("No" at the step ST5), the CPU 6 does not activate the laser-driving circuit 2 to emit the laser light from the semiconductor laser 1, and goes into standby mode ("Yes" at the step ST10 ∼ "No" at the step ST5 ∼ step ST6).

On the other hand, when the touch sensor 14 contacts the painful area S ("Yes" at the step ST10) and the emission button 5 is determined to be switched ON ("Yes" at the step ST5), the CPU 6 activates the laser-driving circuit 2 to emit the laser light from the semiconductor laser 1 (step ST7) . The laser light irradiates the painful area S passing through the irradiation part 10A.

In this way, the CPU 6 determines the contact/noncontact between the touch sensor 14 and the painful area S. When the noncontact is detected, the laser light is not emitted as the irradiation part 10A does not come close to the painful area S. On the other hand, when the contact is detected, the laser light is emitted as the irradiation part 10A comes close to the painful area S under the condition that the emission button 5 is switched ON. Accordingly, it becomes possible to prevent erroneous irradiation so as to avoid accidents causing blindness or the like from occurring.

As described above, the followings are provided according to the third embodiment: the touch sensor 14 placed around the irradiation part 10A which outputs the sensor signal when it contacts the painful area S at the step ST10; the sensor-signal processing circuit 15 which detects the contact between the touch sensor 14 and the painful area S by processing the sensor signal output from the touch sensor 14; and the CPU 6 which activates the laser-driving circuit 2 to emit the laser light from the semiconductor laser 1 under the condition that the emission button 5 is switched ON at the step ST7 only while the sensor-signal processing circuit 15 is sensing the contact with the painful area S. Accordingly, when the irradiation part 10A does not come close to the painful area S, the laser light is not emitted from the semiconductor laser 1 as the touch sensor 14 does not contact the painful area S. Therefore, it becomes possible to prevent erroneous irradiation of the laser light so as to avoid accidents causing blindness or the like, resulting in the safety enhancement of the pain-relief device.

### EMBODIMENT 4

The following embodiment is possible when the painful area S is irradiated with the laser light emitted from the semiconductor laser 1.

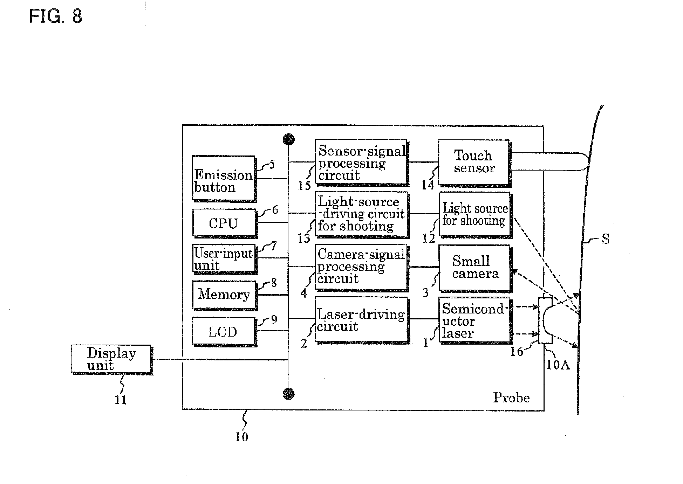

FIG. 8 is a configuration block diagram of the pain-relief device according to the fourth embodiment of the present invention. In FIG. 1 and FIG. 8, a light diffusion lens as a light diffusion mechanism which has a negative refracting power is denoted by 16. In FIG. 8, a planoconcave lens which is cut on a given flat surface including a light axis is illustrated as a light diffusion lens 16.

As the light diffusion lens 16 has a negative refracting power, the laser light emitted from the semiconductor laser 1 at the step ST7 in FIG. 3, FIG. 5 or FIG. 7 is diffused by the light diffusion lens 16 so that the painful area S can be irradiated within a wider range thereof. Therefore, it becomes possible to improve the efficiency of the irradiation and the uniformity of the laser light so that the unevenness of the irradiation can be reduced, resulting in the capability of irradiating the painful area S with the laser light having a milder laser light power.

As described above, according to the fourth embodiment, as the light diffusion lens 16 which has a negative refracting power is further provided and the painful area S is irradiated with the laser light emitted from the semiconductor laser 1 through the light diffusion lens 16 at the step ST7, it becomes possible to diffuse the laser light widely to irradiate the painful area S, resulting in the improvement in efficiency of the irradiation. It also becomes possible to irradiate the painful area S with the uniform laser light, producing the reduction of the unevenness of the irradiation. Furthermore, it also becomes possible to irradiate the painful area S with the laser light having a milder laser light power.

Calculated in terms of a planar dimension, the possible range of the power P of the low-powered heat-producing laser light to irradiate the painful area S is about P≤0.4W/cm² and the wavelength of the low-powered heat-producing laser light is about 600nm≤ λ ≤1000nm.
By irradiating the painful area S with the low-powered laser light by mechanism of the semiconductor laser 1 and the light diffusion lens 16 under the condition of described above, it becomes possible to warm the painful area S and to promote blood circulation, resulting in the relief of pain.

### EXPLANATION OF SYMBOLS

| | |
|---|---|
| 1 | Semiconductor laser |
| 2 | Laser-driving circuit |
| 3 | Small camera |
| 4 | Camera-signal processing circuit |
| 5 | Emission button |
| 6 | CPU |
| 7 | User-input unit |
| 8 | Memory |
| 9 | LCD |
| 10 | Probe |
| 10A | Irradiation part |
| 11 | Display unit |
| 12 | Light source for shooting |
| 13 | Light-source-driving circuit for shooting |
| 14 | Touch sensor |
| 15 | Sensor-signal processing circuit |
| 16 | Light diffusion lens |

## Claims

1. A pain-relief device, comprising:
a laser light source means for emitting heat-producing laser light,
an image-taking means for taking a picture of the target area being irradiated by said laser light source means, and
an image display means for displaying an image of the target area taken by said image-taking means,
and said area which has been imaged by said image-taking means is irradiated with said laser light.

2. The pain-relief device according to claim 1, further comprising:
control means for controlling the laser light source means based on control-relevant information of the laser light source means, and
control information presenting means for presenting said control-relevant information to the user.

3. The pain-relief device according to claim 1 further comprised of an emission request means for utilizing the laser light emission from the laser light source means, and said laser light source means emits the laser light when said emission request means is used.

4. The pain-relief device according to claim 1, wherein the image-taking means further comprises an illuminating means which illuminates the painful area.

5. The pain-relief device according to claim 1, further comprising:
touch sensor means installed around the irradiation part wherein the painful area is irradiated with the laser light for sensing a contact with the painful area; and
control means for controlling the laser light source means to emit the laser light only while said touch sensor means is contacting the painful area.

6. The pain-relief device according to claim 1 further comprised of a light diffusion means which has a negative refracting power and irradiates the painful areas with the laser light emitted from the laser light source means.

7. The pain-relief device according to claim 1, wherein the laser light source means is a semiconductor laser.

8. The pain-relief device according to claim 1, wherein the laser light source means emits the laser light to the target areas of the laser light irradiation within a range of 0.4 W/cm² or less in power.

9. The pain-relief device according to claim 1, wherein the light source means emits the laser light to the target areas of the laser light irradiation within a range from 600nm to 1000nm in wavelength.
